# EUROPEAN PATENT SPECIFICATION

(11) **EP 3 085 692 B1**
(45) Date of publication and mention of the grant of the patent: **21.11.2018**
(21) Application number: 15164350.9
(22) Date of filing: 20.04.2015
(51) Int. Cl.: C07D 219/02

(54) **NOVEL 3,6-DISUBSTITUTED FLUORENE DERIVATIVES**
NEUARTIGE 3,6-DISUBSTITUIERTE FLUORENDERIVATE
NOUVEAUX DÉRIVÉS 3,6-SUBSTITUÉS DE FLUORÈNE

(43) Date of publication of application: 26.10.2016
(73) Proprietor: Nissan Chemical Corporation, Tokyo (JP)
(72) Inventor: Dabeux, Francois, 1140 Evere (BE); Bascour, Doinique, 1390 Grez-Doiceau (BE); Maunoury, Jonathan, 1030 Brussels (BE); Caille, Jean-Raphael, 5000 Namur (BE)
(74) Representative: Dr. Langfinger & Partner

(56) References cited:
- WO-A1-2013/098175
- JP-A- 2006 131 783

## Description

The present invention relates to novel 3,6-disubstituted fluorene derivatives, their use in organic electronic devices and organic electronic devices comprising the novel 3,6-disubstituted fluorene derivatives.

In organic electronic devices, the ability to form morphologically stable amorphous films is a key requirement for the development of small materials for organic light emitting diodes (OLEDs). That is because when a small molecule compound is used in the organic light-emitting layer, crystallization usually occurs if the molecule of the compound is too small and its structure is too symmetrical. Therefore, when applied in an organic emission layer, the small molecule compound is vulnerable to morphological change such as crystallization, and once the crystal is formed, it yields negative impacts upon the light-emitting nature and service life of the OLED.

Organic light-emitting diodes (OLEDs) exploit the property of materials of emitting light when they are excited by electrical current. OLEDs are of particular interest in this regard as an alternative to cathode ray tubes and to liquid-crystal displays for producing flat visual display units.

Due to the compact design and the intrinsically low power consumption compared to classical lighting devices, devices comprising OLEDs are suitable especially for mobile applications, for example for applications in mobile phones, lap-tops, digital cameras, and for illumination purposes.

The basic principles of the way in which OLEDs work and suitable structures (layers) of OLEDs are known to those skilled in the art.

The light-emitting materials (emitters) may be fluorescent materials (fluorescence emitters) or phosphorescent materials (phosphorescence emitters). The phosphorescence emitters have the advantage over fluorescent emitters that they exhibit triplet emission whereas fluorescence emitters only exhibit singlet emission. As a consequence, the quantum efficiency, energy efficiency and power efficiency of devices using phosphorescent emitters may be up to four times as high than for fluorescence emitters.

In order to implement the advantages of the use of the organometallic triplet emitters in practice, it is necessary to provide device compositions which have a high operative lifetime, a high stability to thermal stress and a low use and operating voltage.

Usually, organic light emitting devices comprise several layers to optimize the suitability of the device for the specific intended purpose.

The emissive layer comprises the emissive material and, in many cases a matrix material in which the emissive material is distributed. This material is generally referred to as host material.

Besides the emissive layer organic electronic devices usually comprise so called hole transport layers (HTL), sometimes also referred to as electron blocking layers.

Furthermore, additional layers may be present such as hole injection layers, electron injection layers etc.

For use in hole transport layers of hole injection layers as well as for the use as host materials in emissive layers, a high triplet level of the compounds is desirable. A high triplet level should avoid excitons quenching at the interface of hole transport layer and emissive layer and should thus improve the lifetime and efficiency of the devices.

Fluorene-based materials have been used in the active layer of thin film devices; most of the molecules which have been described in the literature are based on the fluorene-2,7-diyl structure.

Song et al., Tetrahedron Lett. 51(2010), 4894 describe the synthesis and properties of cyclic ethylene-bridged 3,6-fluorene dimers and linear analogues thereof.

WO2013/045410 discloses i.a. compounds of formulae 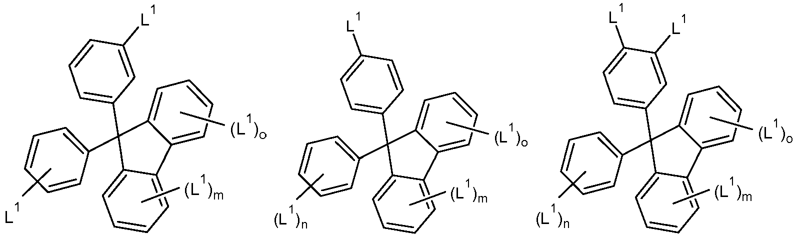 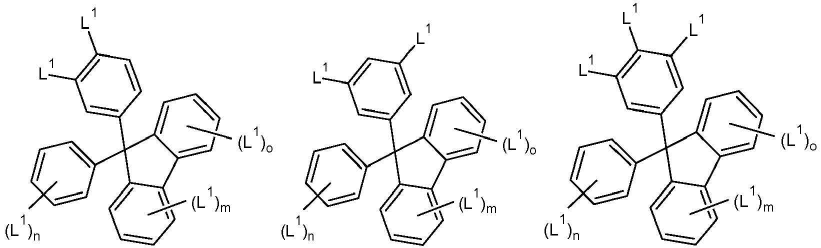 wherein L¹ may i.a. be an acridine derivative, i.e. a substituent the core of which resembles formula (2).

JP-A-2006-131783 discloses materials for organic electroluminescent elements having high luminous efficiency and a long life.

WO-A-2013/098175 discloses crosslinkable arylamine compounds useful for the manufacture of organic electronic devices.

There still exists an ongoing need for compounds with high triplet level useful in organic electronic devices.

It was thus an object of the present invention to provide materials with high triplet level suitable for use in organic electronic devices, in particular in organic light emitting diodes.

This object has been achieved with the compounds in accordance with formula (1).

Preferred embodiments of the present invention are described in the dependent claims and the detailed specification hereinafter.

The compounds in accordance with the present invention are represented by formula (1) 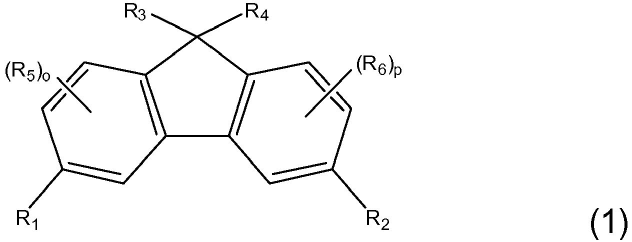 wherein R₁ and R₂, which may be the same or different, represent a substituent of formula (2) 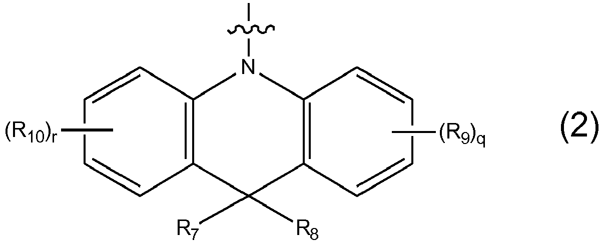 wherein
R₇ and R₈, which may be the same or different, represent hydrogen or an aryl, linear or branched alkyl, cycloalkyl, alkenyl, silyl, nitro, cyano or imino group,
R₉ and R₁₀, which may be the same or different, represent a substituent other than hydrogen, preferably a group as defined above for R₇ and R₈, o, p ,q, r and s, which may be the same or different, are 0, 1, 2 or 3, preferably 0, 1 or 2, even more preferably 0 or 1, most preferably 0, R₃ and R₄, which may be the same or different, represent a group -(CH₂)ₙ-Aryl, -(CH₂)ₙ-heteroaryl, (CH₂)ₙ-Vinyl, -(CH₂)ₙ-Aryl-vinyl, or a cyano, nitro, silyl, C₁-C₁₈-alkyl, C₃-C₂₀-cycloalkyl, ether, thioether or polyether group, wherein
n is 0 or an integer of from 1 to 20, preferably of from 1 to 8, Aryl is a C₅-C₃₀ aryl group and heteroaryl is a C₂-C₃₀ heteroaryl group, and R₅ and R₆, which may be the same or different, represent a substituent other than hydrogen.

If R₅ and or R₆ are present (o and or p different from 0), they are preferably selected from groups as defined above for R₇ and R₈ except hydrogen. In a preferred embodiment, o and p are 0, i.e. the respective rings do not bear substituents other than hydrogen.

Compounds in accordance with claim 1 wherein R₇ and R₈, which may be the same or different, represent a C₁-C₈-alkyl group, particularly preferred a methyl group, are also preferred.

Another preferred class of compounds of formula (1) are those, in which R₃ and R₄, which may be the same or different are represented by a - (CH₂)ₙ-Aryl-Vinyl group, in which Aryl is particularly preferred a phenyl group.

Compounds in which R₃ and R₄ represent an alkyl group and particularly a methyl group, are also preferred.

The term aryl, as used herein, shall mean a C₅-C₃₀ aryl group and for the purposes of the present invention denotes groups having at least one 5 to 7 membered aromatic ring with (4n+2) *π* electrons in accordance with the so called Hückel rule. Two or more aromatic rings may be annealed of fused or may be connected through a bond or an alkyl group with each other.

Representative aryl groups are thus phenyl, biphenyl, naphthyl or anthracenyl.

The aromatic rings in the aryl groups may be un-substituted or substituted by substituents selected from the group consisting of halogen, alkyl, alkoxy, amino, cyano, alkenyl, alkynyl, arylalkyl, aryl and heteroaryl groups.

Particularly preferred aryl groups are C₆-C₁₄ aryl groups like phenyl, biphenyl naphthyl and anthracenyl, which may be substituted or unsubstituted and in some cases substituted or unsubstituted phenyl, in particular unsubstituted phenyl have shown advantageous results.

The term alkyl group, as used herein, denotes a linear, branched or cyclic group comprising 1 to 18 carbon atoms with hydrogen atoms as substituents. Linear or branched alkyl groups having 1 to 12, in particular 1 to 8 carbon atoms are preferred. Linear alkyl groups having 1 to 6 and especially 1 to 4 carbon atoms such as methyl, ethyl, n-propyl, n-butyl, n-pentyl and n-hexyl are especially preferred.

Representative examples of branched alkyl groups with 1 to 8 carbon atoms are i-propyl, i- and t-butyl and the isomeric branched methyl-or ethyl-substituted pentyl- or hexyl derivatives like e.g. 2-methylpentyl, 3-methylpentyl, 2-ethylhexyl, 3-ethylhexyl or 4-ethylhexyl to mention only a few typical representatives.

The term alkenyl group, as used herein, shall mean substituted or unsubstituted linear or branched aliphatic groups with 2 to 18 carbon atoms and at least one carbon-carbon double bond in the main chain. A preferred alkenyl group is the ethenyl group -CH=CH₂ which is also commonly designated as vinyl group. A further preferred group is the 1-propenyl or allyl group -CH₂-CH=CH₂.

The term heteroaryl group, when used herein, denotes a group with 2 to 30 carbon atoms having an aromatic ring comprising at least one heteroatom, which may be substituted or unsubstituted as described above for the aryl groups.

Preferred heteroaryl groups are heteroaryl rings containing at least one donor nitrogen atom. Said rings may be un-substituted or substituted by substituents selected from the group consisting of halogen, alkyl, alkoxy, amino, cyano, alkenyl, alkynyl, arylalkyl, aryl and heteroaryl group and/or may form an annealed ring system with other rings selected from cycloalkyl, aryl and heteroaryl rings. Heteroaryl substituents may be preferably un-substituted or substituted carbazolyl or un-substituted or substituted dibenzofuranyl.

More particularly heteroaryl groups are derived from the heteroarenes group consisting of 2H-pyrrole, 3H-pyrrole, 1H-imidazole, 2H-imidazole, 4H-imidazole, 1H-1,2,3-triazole, 2H-1,2,3-triazole, 1H-1,2,4-triazole, 1H-pyrazole, 1H-1,2,3,4-tetrazole, imidazol-2-ylidene, oxazole, isoxazole, thiazole, isothiazole, 1,2,3-oxadiazole, 1,2,5-oxadiazole, 1,2,3- thiadiazole and 1,2,5-thiadazole rings.

Nitrogen containing heteroaryl groups are preferably derived from the heteroarenes shown below 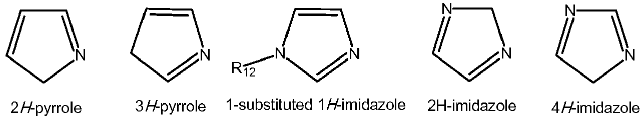 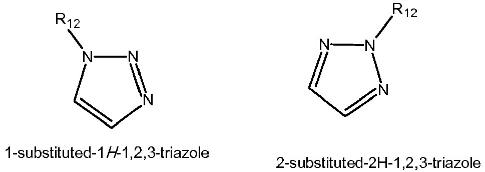 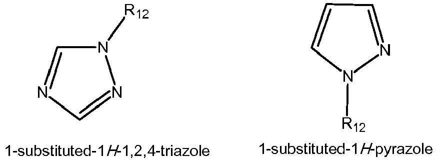 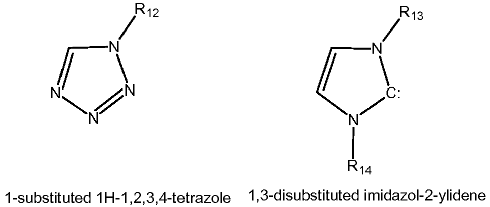 wherein R₁₂, R₁₃ and R₁₄ may be selected from a broad variety of substituents such as alkyl, alkenyl, alkynyl, arylalkyl, aryl and heteroaryl groups.

Further preferred heteroaryl groups are derived from the following heteroarenes 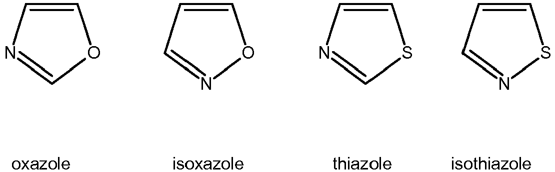 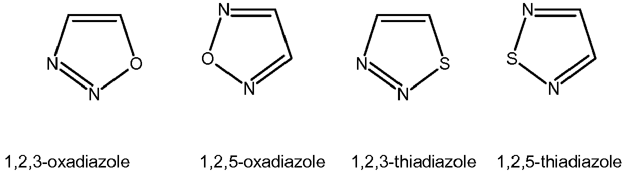 wherein the rings can be unsubstituted or substituted with one or more substituents.

Still another preferred group of heteroaryl substituents comprises the 6-membered ring systems shown below:

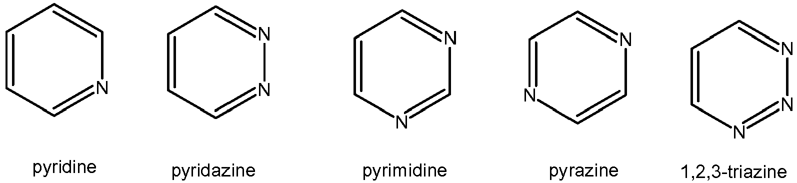

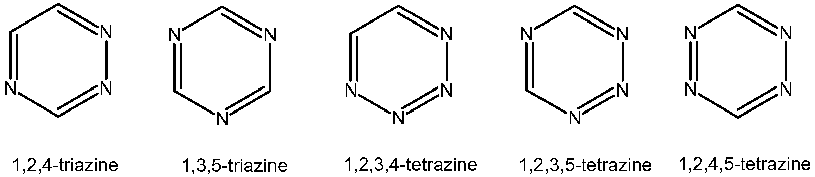

The heteroaryl groups may form or be part of an annealed ring system where several rings are condensed or annealed.

The term cycloalkyl, as used herein, is used for a carbocyclic group with 3 to 20 carbon atoms, which may be substituted or unsubstituted. Representative examples are substituted or unsubstituted cycloalkyl groups with 5 to 6 carbon atoms and preferably 5 to 7 carbon atoms in the ring.

The optional substituents of the aforementioned aryl, heteroaryl or cycloalkyl groups, are preferably selected from aliphatic groups, carbocyclic groups, aromatic groups or heterocyclic groups, oxo, OR¹⁵, NR¹⁶R¹⁷ and SR¹⁸ groups, wherein R¹⁵ to R¹⁸ are the same or different and represent hydrogen, an aliphatic group, a carbocyclic group, an aromatic group or a heterocyclic group having 1 to 20 carbon atoms.

In accordance with still another preferred embodiment, the substituents of the aliphatic, carbocyclic, aromatic or heteroaromatic groups are selected from the group consisting of halogen, alkyl, alkoxy, aryloxy, oxo, amino, substituted amino, cyano, alkenyl, alkynyl, arylalkyl, aryl and heteroaryl groups, even more preferably from halogen, alkyl, alkoxy, amino, cyano, alkenyl, alkynyl, arylalkyl, aryl and heteroaryl groups.

The variables o, p and q, which may be the same or different, are each 0, 1, 2 or 3, preferably 0, 1 or 2 and in some cases compounds in which o, p and q are 0 have shown advantageous properties.

Compounds (4) to (6) shown below represent a group of particularly preferred compounds

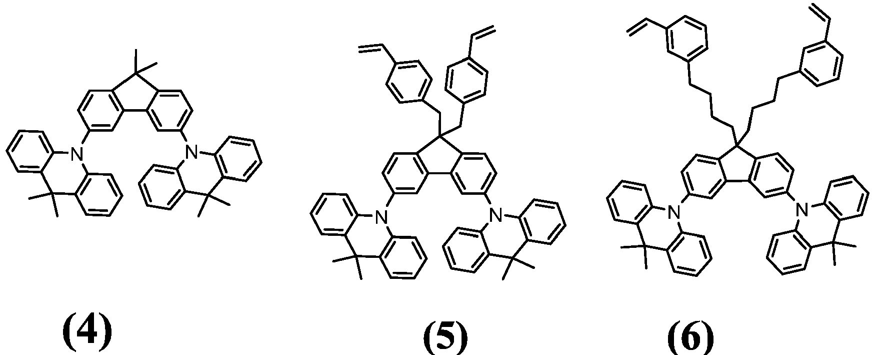

The compounds in accordance with the present invention usually have a triplet level exceeding 2.8 eV and are therefore particularly suitable for use in organic electronic devices, in particular organic light emitting diode (OLED).

Accordingly, another embodiment of the present invention is directed to organic electronic devices, in particular organic light emitting diodes, comprising a compound in accordance with the present invention, preferably in the hole transport layer, the hole injection layer or as a host material in the emissive layer.

An OLED generally comprises :
a substrate, for example (but not limited to) glass, plastic, metal;
an anode, generally transparent anode, such as an indium-tin oxide (ITO) anode;
a hole injection layer (HIL) for example (but not limited to) PEDOT/PSS;
a hole transporting layer (HTL);
an emissive layer (EML);
an electron transporting layer (ETL);
an electron injection layer (EIL) such as LiF, Cs₂CO₃
a cathode, generally a metallic cathode, such as an Al layer.

For a hole conducting emissive layer, one may have a hole blocking layer (HBL) that can also act as an exciton blocking layer between the emissive layer and the electron transporting layer. For an electron conducting emissive layer, one may have an electron blocking layer (EBL) that can also act as an exciton blocking layer between the emissive layer and the hole transporting layer. The emissive layer may be equal to the hole transporting layer (in which case the exciton blocking layer is near or at the anode) or to the electron transporting layer (in which case the exciton blocking layer is near or at the cathode).

Due to their high triplet level, the compounds of the present invention may help to avoid exciton quenching at the interface e.g. of HTL and EML which improves efficiency and lifetime of the devices.

The compounds in accordance with the present invention may be synthesized using processes known to the skilled person, who will select the appropriate process, reactants and reactant conditions based on his professional knowledge and adopted to the specific compound to be synthesized.

Generally said, the compounds of the present invention, from a retrosynthesis point of view, can be synthesized from a common precursor, 3,6-dibromofluorenone, which is commercially available or may be synthesized in a manner known to the skilled person.

Generally, 3,6-dibromofluorenone is reduced to its fluorene analogue before introduction of substituents R₃ and R₄ by a suitable reagent. A subsequent Buchwald-Hartwig coupling with the respective reactants providing groups R₁ and R₂ then yields the final product.

An exemplary reaction scheme for the synthesis of preferred compounds of formula (4) and (5) is the following:

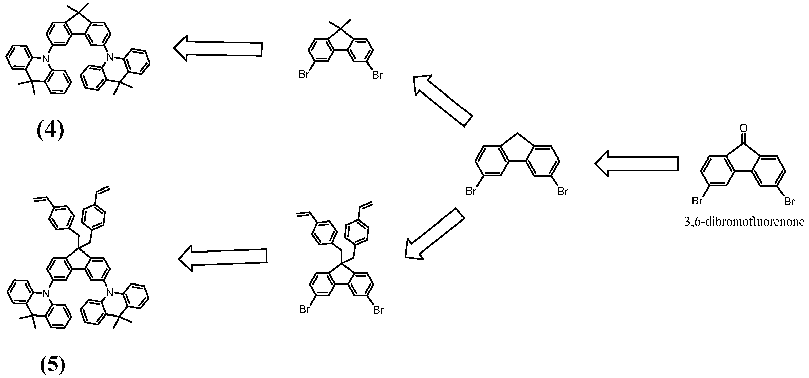

The reduction of the dibromofluorenone in accordance with the following exemplary scheme 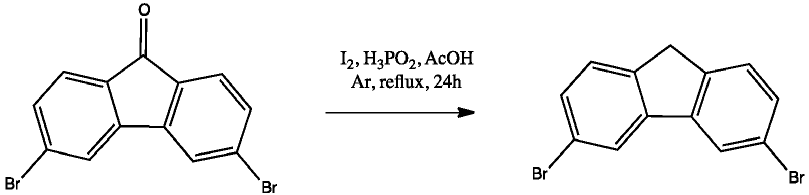 has been extensively described in the literature. Traditional methods are well known as Wolf-Kishner and Clemmensen reductions and suitable conditions are i.a.described by Hicks and coworkers (Hicks, L. ; Han, J. K. ; Fry, A. J. ; Tetrahedron Lett., 2000, 41, 7817-7820) to which reference is made for further details.

Introducing substituents R₃ and R₄ is exemplary shown in the following scheme for alkyl groups, which represent a preferred group of substituents in the compounds in accordance with the present invention.

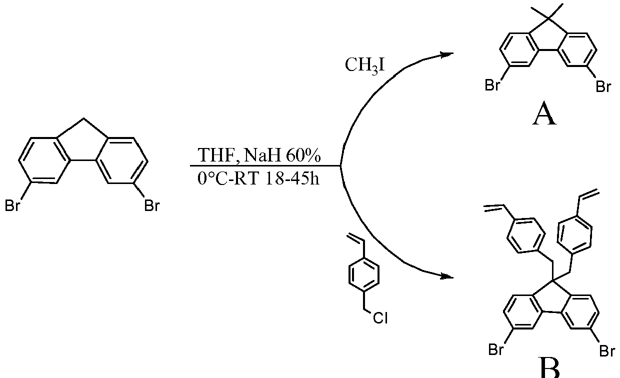

The final step is then preferably a so-called Buchwald-Hartwig coupling or other suitable coupling method with which substituents R₁ and R₂ are introduced to obtain the desired product.

Suitable carbon-carbon coupling methods have been described extensively in the literature and are known to the skilled person so that no further details need to be given here.

### Examples: Synthesis of compounds of formula (4) and (5)

### Example 1 - Reduction of dibromofluorenone

5,92g (23.3 mmoles, 0.5eq) I₂, 190mL of acetic acid and 12mL (111 mmoles, 2.6eq) H₃PO₂ (50% aq) were introduced in a three necked flask and heated to reflux. A solution of 15,15g of 3,6-dibromofluorenone in 150mL acetic acid were added to the mixture and heated to reflux for 4 hours, cooled down to room temperature, diluted with 400mL water, dissolved with 500 mL toluene and extracted. The aqueous phase was extracted twice with 400 mL toluene. The organic phase was washed 3 times with water (500 mL), dried on MgSO₄ and filtered before evaporation. 17.70g of a mixture of 3,6-dibromofluorene and its tribromo derivative were obtained. The yield obtained for the reduction is 99%.

### Example 2 - Alkylation of the 3,6-dibromofluorene

4,69 g of 3,6- dibromofluorene (11,2 mmoles) were dissolved in 44 mL of anhydrous THF under inert atmosphere and cooled down to 0°C. 1,53g of NaH 60% in mineral oil (38 mmoles, 3 eq) were then added and after 10 minutes, 2.40 mL of CH₃I (38 mmoles, 3 eq) were added. After 18h at room temperature, the medium was quenched with 50mL of water and extracted three times with ethyl acetate (50 mL). The organic phase was washed with water (3 X 70 mL), dried on MgSO₄ and evaporated. The purification by chromatography onto silica (2* 240g) with pure hexane as eluent gave 2,01g (5.7 mmoles) of expected product 3,6-dibromo-9,9-dimethylfluorene with 51% yield (NMR purity: 98%).

### Example 3 - Buchwald-Hartwig coupling for the synthesis of compound of formula (4)

3,15 g of 3,6-dibromo-9,9-dimethylfluorene(8,7 mmol), 3,98g of 9,9-dimethylacridine (19 mmol, 2,2 eq) and 4,37g of NaOtBu (45,5 mmol, 5,2 eq) were dissolved in 90mL of anhydrous toluene and the medium was degassed with argon for 30 minutes before adding the catalytic system (0,32g Pd(dba)₂ (0,564mmol, 0,06 eq) in 30mL of anhydrous toluene). and 1,10mL P(tBu)₃ 1M in toluene (1,1mmol, 0,13 eq). The medium was heated to reflux for 18 hours and then cooled down and plugged on 100g silica. 5.86g of the compound of formula (4) were obtained with 88% NMR purity and 95% yield.

### Example 3 - Buchwald-Hartwig coupling for the synthesis of compound of formula (5)

2.98g of 3,6-dibromo-9,9-divinylbenzylfluorene(5.0 mmol), 2.20g of 9,9-dimethylacridine (10.5 mmol, 2,1eq) and 2.39g of NaOtBu (24.8 mmol, 5 eq) were dissolved in 100mL of anhydrous toluene and the medium was degassed with argon for 30 minutes before adding the catalytic system (0,17g Pd(dba)₂ (0,31mmol, 0,06 eq) in 20mL of anhydrous toluene) and 0.6mL P(tBu)₃ 1M in toluene (0.6 mmol, 0,12 eq). The medium was heated at 80°C for 22 hours and then cooled down and extracted three times (toluene/water). The organic phase was then washed with water, dried over MgSO₄, filtered and evaporated to afford 2.49g of the compound of formula (5) (60% NMR purity) and 37% yield.

## Claims

1. Compounds of formula (1) 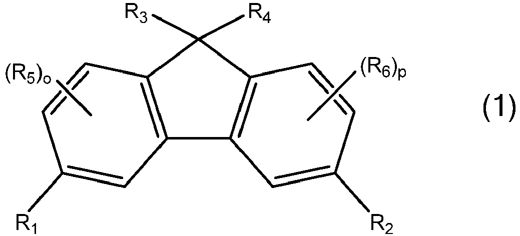 wherein R₁ and R₂, which may be the same or different, represent a substituent of formula (2) 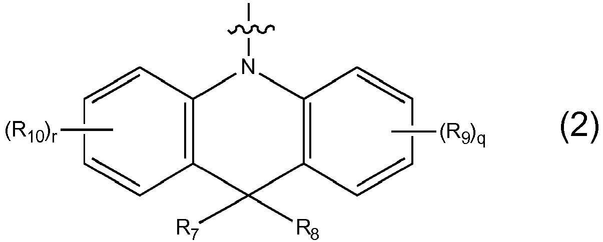 wherein
R₇ and R₈, which may be the same or different, represent hydrogen or an aryl, linear or branched alkyl, cycloalkyl, alkenyl, silyl, nitro, cyano or imino group, R₉ and R₁₀, which may be the same or different, represent a substituent other than hydrogen
o, p ,q, r and s, which may be the same or different, are 0, 1, 2 or 3,
R₃ and R₄, which may be the same or different, represent a group -(CH₂)ₙ-Aryl, -(CH₂)ₙ-heteroaryl, (CH₂)ₙ-vinyl, -(CH₂)ₙ-Aryl-vinyl, or a cyano, nitro, silyl, C₁-C₁₈-alkyl, C₃-C₂₀-cycloalkyl, ether, thioether or polyether group, wherein n is 0 or an integer of from 1 to 20, Aryl is a C₅-C₃₀ aryl group and heteroaryl is a C₂-C₃₀ heteroaryl group,
R₅ and R₆, which may be the same or different, represent a substituent other than hydrogen,
aromatic rings in the aryl and heteroaryl groups and the carbocyclic group of the cycloalkyl groups may be un-substituted or substituted by substituents, and
said optional substituents of aryl, heteroaryl and cycloalkyl groups are selected from aliphatic groups, carbocyclic groups, aromatic groups or heterocyclic groups, oxo, OR15, NR¹⁶R¹⁷ and SR¹⁸ groups, wherein R¹⁵ to R¹⁸ are the same or different and represent hydrogen, an aliphatic group, a carbocyclic group, an aromatic group or a heterocyclic group having 1 to 20 carbon atom and/or from the group consisting of halogen, alkyl, alkoxy, aryloxy, oxo, amino, substituted amino, cyano, alkenyl, alkynyl, arylalkyl, aryl and heteroaryl groups.

2. Compounds in accordance with claim 1 wherein R₇ and R₈, which may be the same or different, represent a C₁-C₈-alkyl group wherein the term "alkyl" denotes a linear, branched or cyclic group comprising 1 to 18 carbon atoms with hydrogen atoms as substituents.

3. Compounds in accordance with claim 1 or 2 wherein R₃ and R₄, which may be the same or different are represented by a -(CH₂)ₙ-Aryl-Vinyl group.

4. Compounds in accordance with claim 3 wherein the aryl group is a phenyl group.

5. Compounds in accordance with any of claim 1 or 2 wherein R₃ and R₄, which may be the same or different are represented by an alkyl group wherein the term "alkyl" denotes a linear, branched or cyclic group comprising 1 to 18 carbon atoms with hydrogen atoms as substituents.

6. Compounds in accordance with claim 5 wherein the alkyl group is a methyl group.

7. Compounds in accordance with claim 1 represented by formulae (4) to (6)

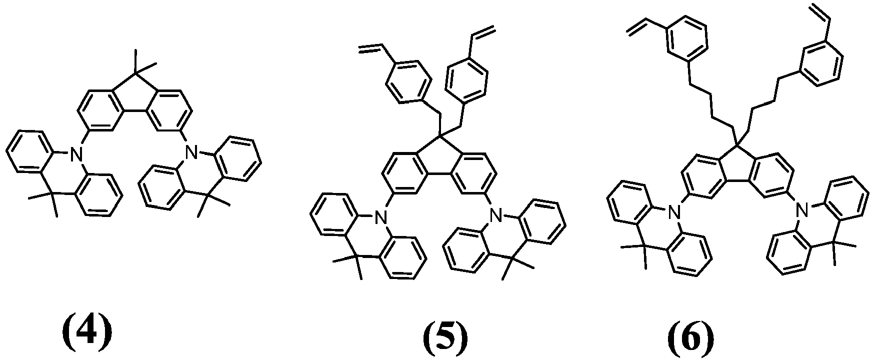

8. Use of the compounds in accordance with any of claims 1 to 7 in organic electronic devices.

9. Use in accordance with claim 8 wherein the organic electronic device is an organic light emitting diode (OLED).

10. Organic electronic device comprising a compound in accordance with any of claims 1 to 7.

11. Organic electronic device in accordance with claim 10 which is an organic light emitting diode (OLED).

12. Organic light emitting diode in accordance with claim 11 comprising a compound in accordance with any of claims 1 to 7 in the hole transport layer, and/or the hole injection layer.

13. Organic light emitting diode in accordance with claim 11 comprising a compound in accordance with any of claims 1 to 7 as host material in an emissive layer.

## Patentansprüche

1. Verbindungen der Formel (1) 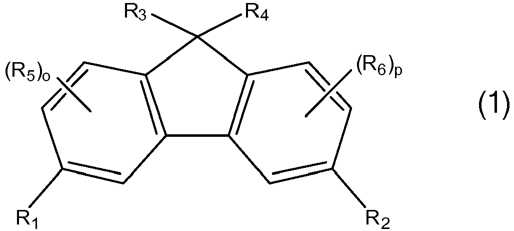 wobei R₁ und R₂, die gleich oder verschieden sein können, für einen Substituenten der Formel (2) 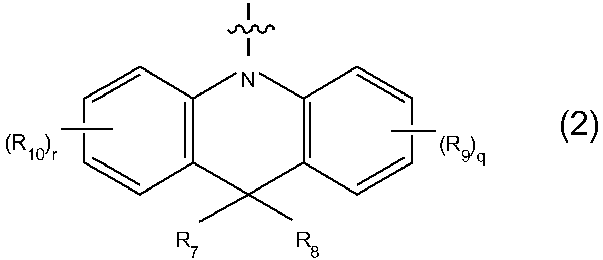 stehen, wobei
R₇ und R₈, die gleich oder verschieden sein können, für Wasserstoff oder eine Aryl-, lineare oder verzweigte Alkyl-, Cycloalkyl-, Alkenyl-, Silyl-, Nitro-, Cyano- oder Iminogruppe stehen,
R₉ und R₁₀, die gleich oder verschieden sein können, für einen von Wasserstoff verschiedenen Substituenten stehen,
o, p, q, r und s, die gleich oder verschieden sein können, für 0, 1, 2 oder 3 stehen,
R₃ und R₄, die gleich oder verschieden sein können, für eine Gruppe -(CH₂)ₙ-Aryl, -(CH₂)ₙ-Heteroaryl, (CH₂)ₙ-Vinyl, -(CH₂)ₙ-Aryl-vinyl oder eine Cyano-, Nitro-, Silyl-, C₁-C₁₈-Alkyl-, C₃-C₂₀-Cycloalkyl-, Ether-, Thioether- oder Polyethergruppe stehen, wobei n für 0 oder eine ganze Zahl von 1 bis 20 steht, Aryl für eine C₅-C₃₀-Arylgruppe steht und Heteroaryl für eine C₂-C₃₀-Heteroarylgruppe steht, R₅ und R₆, die gleich oder verschieden sein können, für einen von Wasserstoff verschiedenen Substituenten stehen,
aromatische Ringe in den Aryl- und Heteroarylgruppen und die carbocyclische Gruppe der Cycloalkylgruppen unsubstituiert oder durch Substituenten substituiert sein können, und
wobei die fakultativen Substituenten von Aryl-, Heteroaryl- und Cycloalkylgruppen aus aliphatischen Gruppen, carbocyclischen Gruppen, aromatischen Gruppen oder heterocyclischen Gruppen, Oxo-, OR¹⁵-, NR¹⁶R¹⁷- und SR¹⁸-Gruppen, wobei R¹⁵ bis R¹⁸ gleich oder verschieden sind und für Wasserstoff, eine aliphatische Gruppe, eine carbocyclische Gruppe, eine aromatische Gruppe oder eine heterocyclische Gruppe mit 1 bis 20 Kohlenstoffatomen stehen, und/oder aus der Gruppe bestehend aus Halogen-, Alkyl-, Alkoxy-, Aryloxy-, Oxo-, Amino-, substituierten Amino-, Cyano-, Alkenyl-, Alkinyl-, Arylalkyl-, Aryl- und Heteroarylgruppen ausgewählt sind.

2. Verbindungen nach Anspruch 1, wobei R₇ und R₈, die gleich oder verschieden sein können, für eine C₁-C₈-Alkylgruppe stehen, wobei der Begriff "Alkyl" eine lineare, verzweigte oder cyclische Gruppe mit 1 bis 18 Kohlenstoffatomen mit Wasserstoffatomen als Substituenten bedeutet.

3. Verbindungen nach Anspruch 1 oder 2, wobei R₃ und R₄, die gleich oder verschieden sein können, durch eine -(CH₂)ₙ-Aryl-vinyl-Gruppe wiedergegeben werden.

4. Verbindungen nach Anspruch 3, wobei es sich bei der Arylgruppe um eine Phenylgruppe handelt.

5. Verbindungen nach Anspruch 1 oder 2, wobei R₃ und R₄, die gleich oder verschieden sein können, durch eine Alkylgruppe wiedergegeben werden, wobei der Begriff "Alkyl" eine lineare, verzweigte oder cyclische Gruppe mit 1 bis 18 Kohlenstoffatomen mit Wasserstoffatomen als Substituenten bedeutet.

6. Verbindungen nach Anspruch 5, wobei es sich bei der Alkylgruppe um eine Methylgruppe handelt.

7. Verbindungen nach Anspruch 1, die durch die Formeln (4) bis (6) wiedergegeben werden:

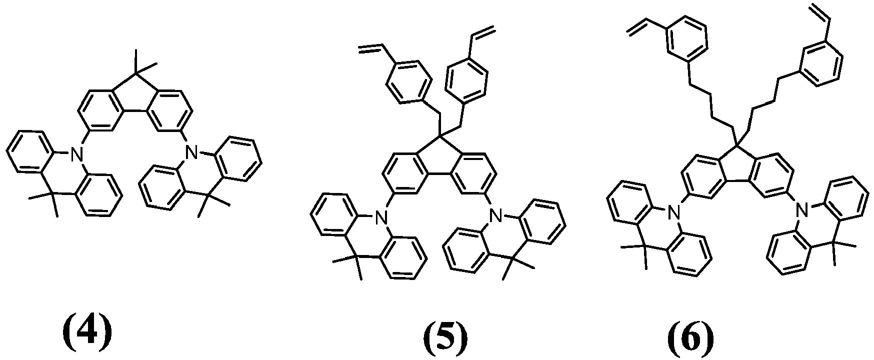

8. Verwendung der Verbindungen nach einem der Ansprüche 1 bis 7 in organischen elektronischen Vorrichtungen.

9. Verwendung nach Anspruch 8, wobei sich bei der organischen elektronischen Vorrichtung um eine organische Leuchtdiode (OLED) handelt.

10. Organische elektronische Vorrichtung, umfassend eine Verbindung nach einem der Ansprüche 1 bis 7.

11. Organische elektronische Vorrichtung nach Anspruch 10, bei der es sich um eine organische Leuchtdiode (OLED) handelt.

12. Organische Leuchtdiode nach Anspruch 11, umfassend eine Verbindung nach einem der Ansprüche 1 bis 7 in der Lochtransportschicht und/oder der Lochinjektionsschicht.

13. Organische Leuchtdiode nach Anspruch 11, umfassend eine Verbindung nach einem der Ansprüche 1 bis 7 als Wirtsmaterial in einer Emissionsschicht.

## Revendications

1. Composés de formule (1) 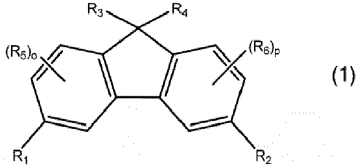 dans laquelle R₁ et R₂, qui peuvent être identiques ou différents, représentent un substituant de formule (2) 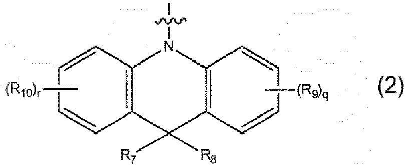 dans laquelle
R₇ et R₈, qui peuvent être identiques ou différents, représentent un hydrogène ou un groupe aryle, alkyle linéaire ou ramifié, cycloalkyle, alcényle, silyle, nitro, cyano ou imino,
R₉ et R₁₀, qui peuvent être identiques ou différents, représentent un autre substituant que l'hydrogène,
o, p, q, r et s, qui peuvent être identiques ou différents, valent 0, 1, 2 ou 3,
R₃ et R₄, qui peuvent être identiques ou différents, représentent un groupe -(CH₂)ₙ-aryle,-(CH₂)ₙ-hétéroaryle, (CH₂)ₙ-vinyle, -(CH₂)ₙ-aryl-vinyle, ou un groupe cyano, nitro, silyle, alkyle en C₁-C₁₈, cycloalkyle en C₃-C₂₀, éther, thioéther ou polyéther, dans lesquels n vaut 0 ou est un entier de 1 à 20, l'aryle est un groupe aryle en C₅-C₃₀ et l'hétéroaryle est un groupe hétéroaryle en C₂-C₃₀,
R₅ et R₆, qui peuvent être identiques ou différents, représentent un autre substituant que l'hydrogène,
les cycles aromatiques dans les groupes aryle et hétéroaryle et le groupe carbocyclique des groupes cycloalkyle peuvent être non substitués ou substitués par des substituants, et
lesdits substituants facultatifs des groupes aryle, hétéroaryle et cycloalkyle sont choisis parmi les groupes aliphatiques, les groupes carbocycliques, les groupes aromatiques ou les groupes hétérocycliques, les groupes oxo, OR¹⁵, NR¹⁶R¹⁷ et SR¹⁸, dans lesquels R¹⁵ à R¹⁸ sont identiques ou différents et représentent l'hydrogène, un groupe aliphatique, un groupe carbocyclique, un groupe aromatique ou un groupe hétérocyclique ayant 1 à 20 atomes de carbone et/ou dans le groupe constitué par les groupes halogéno, alkyle, alcoxy, aryloxy, oxo, amino, amino substitués, cyano, alcényle, alcynyle, arylalkyle, aryle et hétéroaryle.

2. Composés selon la revendication 1 dans lesquels R₇ et R₈, qui peuvent être identiques ou différents, représentent un groupe alkyle en C₁-C₈, le terme « alkyle » désignant un groupe linéaire, ramifié ou cyclique comprenant 1 à 18 atomes de carbone avec des atomes d'hydrogène comme substituants.

3. Composés selon la revendication 1 ou 2 dans lesquels R₃ et R₄, qui peuvent être identiques ou différents, sont représentés par un groupe -(CH₂)ₙ-aryl-vinyle.

4. Composés selon la revendication 3 dans lesquels le groupe aryle est un groupe phényle.

5. Composés selon la revendication 1 ou 2 dans lesquels R₃ et R₄, qui peuvent être identiques ou différents, sont représentés par un groupe alkyle, le terme « alkyle » désignant un groupe linéaire, ramifié ou cyclique comprenant 1 à 18 atomes de carbone avec des atomes d'hydrogène comme substituants.

6. Composés selon la revendication 5 dans lesquels le groupe alkyle est un groupe méthyle.

7. Composés selon la revendication 1 représentés par les formules (4) à (6)

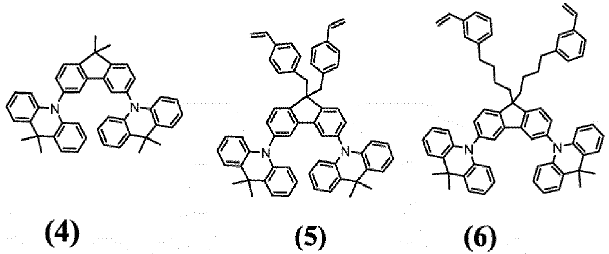

8. Utilisation des composés selon l'une quelconque des revendications 1 à 7 dans des dispositifs électroniques organiques.

9. Utilisation selon la revendication 8 dans lequel le dispositif électronique organique est une diode électroluminescente organique (OLED).

10. Dispositif électronique organique comprenant un composé selon l'une quelconque des revendications 1 à 7.

11. Dispositif électronique organique selon la revendication 10 qui est une diode électroluminescente organique (OLED).

12. Diode électroluminescente organique selon la revendication 11 comprenant un composé selon l'une quelconque des revendications 1 à 7 dans la couche de transport de trous, et/ou la couche d'injection de trous.

13. Diode électroluminescente organique selon la revendication 11 comprenant un composé selon l'une quelconque des revendications 1 à 7 comme matériau hôte dans une couche émissive.
